# EUROPÄISCHE PATENTSCHRIFT

(11) **EP 3 350 151 B1**
(45) Veröffentlichungstag und Bekanntmachung des Hinweises auf die Patenterteilung: **13.11.2019**
(21) Anmeldenummer: 16770909.6
(22) Anmeldetag: 19.09.2016
(51) Int. Cl.: C07C 63/24, C07C 51/41, C07F 5/06, C07F 15/02, B01J 20/22, B01J 20/28

(54) **VERFAHREN ZUR HERSTELLUNG VON METALLORGANISCHEN GERÜSTSTRUKTURVERBINDUNGEN**
METHOD FOR PRODUCING METAL-ORGANIC FRAMEWORKS
PROCÉDÉ DE FABRICATION DE RÉSEAUX ORGANOMÉTALLIQUES

(30) Priorität: 17.09.2015 DE 102015115738
(43) Veröffentlichungstag der Anmeldung: 25.07.2018
(73) Patentinhaber: Christian-Albrechts-Universität zu Kiel, 24118 Kiel (DE); Fraunhofer-Gesellschaft zur Förderung der angewandten Forschung e.V., 80686 München (DE)
(72) Erfinder: FRÖHLICH, Dominik, 79115 Freiburg (DE); HOLZ, Albina, 79110 Freiburg (DE); HENNINGER, Stefan, 79346 Endingen a. K. (DE); LENZEN, Dirk, 24896 Treia (DE); REINSCH, Helge, 24106 Kiel (DE); STOCK, Norbert, 24118 Kiel (DE)
(74) Vertreter: Fuchs Patentanwälte Partnerschaft mbB
(86) Internationale Anmeldenummer: PCT/EP2016/072152
(87) Internationale Veröffentlichungsnummer: WO 2017/046417

(56) Entgegenhaltungen:
- WO-A1-2013/186542
- DE-A1-102005 039 623
- DE-A1-102006 043 648
- DE-A1-102009 027 821
- AMANDINE CADIAU ET AL: "Design of Hydrophilic Metal Organic Framework Water Adsorbents for Heat Reallocation", ADVANCED MATERIALS, Bd. 27, Nr. 32, 1. August 2015 (2015-08-01), Seiten 4775-4780, XP055322406, DE ISSN: 0935-9648, DOI: 10.1002/adma.201502418
- HELGE REINSCH ET AL: "Structures, Sorption Characteristics, and Nonlinear Optical Properties of a New Series of Highly Stable Aluminum MOFs", CHEMISTRY OF MATERIALS, Bd. 25, Nr. 1, 8. Januar 2013 (2013-01-08) , Seiten 17-26, XP055321510, US ISSN: 0897-4756, DOI: 10.1021/cm3025445 in der Anmeldung erwähnt

## Beschreibung

Die vorliegende Erfindung betrifft ein Verfahren zur Herstellung einer metallorganischen Gerüststrukturverbindung.

Adsorptionswärmespeicher bieten die Möglichkeit einer nahezu verlustfreien Speicherung von Wärme, insbesondere im Temperaturbereich bis 250 °C, über lange Zeiträume. Ein Bedarf an solchen Langzeit-Wärmespeichern besteht insbesondere im Zusammenhang mit der solarthermischen Gebäudeheizung in Erdregionen mit starker jahreszeitlicher Schwankung der solaren Einstrahlung, d. h. in allen äquatorfernen Regionen. Hier fällt im Jahresverlauf das größte Angebot der Solarwärme aus thermischen Kollektoren in den Sommer, der Heizwärmebedarf jedoch überwiegend in den Winter. Im Sinne des Aufbaus einer nachhaltigen Energieversorgung, die verstärkt auf erneuerbare Energiequellen setzt, ist die saisonale Wärmespeicherung für die Gebäudeheizung wünschenswert und ist Voraussetzung zur Erreichung hoher solarer Deckungsanteile bei der solarthermischen Gebäudeheizung.

Die Wärmespeicherung im Temperaturbereich bis ca. 250 °C ist auch für viele andere Anwendungen ein wichtiges Thema. So besteht z. B. bei der dezentralen Stromerzeugung in Anlagen mit Kraft-Wärme-Kopplung (KWK) typischerweise das Problem unterschiedlicher zeitlicher Bedarfsprofile für Strom und Wärme. Um diese Anlagen stromgeführt betreiben zu können und die erzeugte Wärme nutzen zu können, muss diese Wärme zwischengespeichert werden, bis sie gebraucht wird. Dazu werden Wärmespeicher mit hoher Energiedichte und hoher Effizienz, d. h. geringen Wärmeverlusten, benötigt.

Adsorptionswärmespeicher haben sich trotz jahrzehntelanger Forschungsanstrengungen bisher nicht am Markt durchgesetzt. Es fehlte bisher vor allem an Adsorptionsmaterialien, die im gewünschten Temperaturbereich einen großen Beladungs- und Wärmeumsatz zeigen. Die vielfach für Wärmespeicheranwendungen untersuchten und eingesetzten Zeolithe, z. B. Zeolithe mit den Strukturtypen LTA und FAU, insbesondere die kommerziell erhältlichen Zeolithe A, X und Y, erfordern typischerweise zur Desorption eine treibende Temperaturdifferenz von mindestens 100 °C zwischen Adsorber und Kondensator, also bei einer Kondensatortemperatur von 35 °C eine Desorptionstemperatur von mindestens 135 °C. Diese Temperatur kann mit typischen Flachkollektoren nicht oder nur bei sehr geringer Kollektoreffizienz erreicht werden. Es werden daher teurere Vakuumröhrenkollektoren oder strahlungskonzentrierende Kollektoren benötigt. Unter typischen Be- und Entladebedingungen eines saisonalen solaren Speichersystems, wie z. B. beschrieben in Mittelbach et al., "Solid sorption thermal energy storage for solar heating Systems" (TERRASTOCK 2000, Stuttgart, 28.8. - 1.9.2000), werden mit den genannten Zeolithen Beladungsumsätze von nicht mehr als 0,18 Gramm Wasser pro Gramm Zeolith erreicht. Bezogen auf die Dichte einer Schüttung des Zeoliths sind damit Speicher-Energiedichten bis etwa 150 kWh/m³ erreichbar (A. Hauer, Dissertation, TU Berlin 2002, "Beurteilung fester Adsorbentien in offenen Sorptionssystemen für energetische Anwendungen*"*)*.*

Mit Silikagelen werden vergleichbare Energiedichten erreicht, hier ist das Hauptproblem der geringe nutzbare Temperaturhub bei der Entladung des Speichers.

Für die saisonale solare Wärmespeicherung wird daher nach Adsorbentien gesucht, deren Wasser-Adsorptionseigenschaften zwischen denen typischer Zeolithe und typischer Silikagele liegen. Insbesondere werden Materialien gesucht, deren Adsorptionsisobaren zu einem Wasserdampfdruck von etwa 56 hPa (entsprechend einem Wasserreservoir bei 35 °C) im Temperaturbereich von etwa 60-110 °C eine Beladungsänderung von mindestens 0,2 g/g zeigen.

Metallorganische Gerüstsubstanzen (Metal-Organic Frameworks, MOFs) wurden im Hinblick auf einen möglichen Einsatz als Hochtemperatur-Wasserstoffspeicher oder generell zur sorptiven Gasspeicherung entwickelt (U. Müller, "Metal-organic frameworks-prospective industrial applications", J. Mater. Chem. 16(2006), S. 626-636). Aufgrund der hohen Porosität und Oberfläche eignen sie sich für vielfältige weitere Einsatzgebiete, die klassischerweise durch Zeolithe abgedeckt werden, wie etwa die heterogene Katalyse oder zur Gasreinigung.

MOFs zeichnen sich durch einen modularen Aufbau aus. Sie bestehen aus anorganischen mehrkernigen Komplexen (Cluster), welche als Konnektoren im Netzwerk dienen. Die Zähligkeit und Topologie des Konnektors wird dabei durch die nach außen gerichteten koordinierenden Liganden bestimmt. Als verknüpfende Bausteine (Linker) werden bi-, tri- und multifunktionale Liganden eingesetzt.

Insbesondere MOFs auf Basis von Aluminium als Metallcluster sind hinsichtlich der technischen Verwendung aufgrund des gut verfügbaren und nicht toxischen Metalls sehr vielversprechend. Für viele Anwendungen ist jedoch die geringe Stabilität gegenüber Wasser und insbesondere Wasserdampf hinderlich.

Beispielsweise ist bei der Methanspeicherung eine Restfeuchte im großtechnischen Maßstab nicht zu verhindern. Für die Verwendung in Wärmepumpen und Kältemaschinen auf Basis der Adsorption von Kältemitteln wie beispielsweise Wasser aber auch Alkoholen oder natürlichen Kältemitteln (Propan etc.) wird ebenfalls eine Stabilität gegenüber Wasserdampf vorausgesetzt.

Während sich die Stabilität gegenüber Wasser bei der Verwendung von Wasser als Kältemittel direkt ergibt, ist die Stabilität gegenüber Wasser aber auch bei den anderen Kältemitteln von Bedeutung, da beispielsweise in einigen Prozessschritten der Kontakt zu Wasserdampf (Luftfeuchte bei der Herstellung) nicht verhindert werden kann. Hierbei zeichnet sich beispielsweise das MOF CAU-10-H als sehr vielversprechend ab, da dieser eine hohe Stabilität bei gleichzeitig guter Adsorptionscharakteristik aufweist.

Für die Synthese von MOFs gibt es unterschiedliche Möglichkeiten; die meisten MOFs sind durch Solvothermalsynthesen zugänglich. Dabei werden ein Metallsalz und eine organische Verbindung in einem Lösungsmittel oder Lösungsmittelgemisch suspendiert und das Reaktionsgemisch in einem Druckreaktor erhitzt. Dies ist auch die in der Literatur zu findende, gängige Synthese für CAU-10-H (H. Reinsch, M. A. van der Veen, B. Gil, B. Marszalek, T. Verbiest, D. de Vos and N. Stock, Chemistry of Materials, 2013, 25, 17-26): Als Reaktionsgemisch dient eine Suspension von Isophthalsäure (1,3-H₂BDC) und Al₂(SO₄)₃*18 H₂O in DMF und Wasser (1:4 Teile). Die Synthese wird in einem Autoklaven mit Teflon-Linern über 12 h bei 135 °C ausgeführt. Es wird berichtet, dass bei der Synthese in einem Glasreaktor eine unbekannte, kristalline Nebenphase erhalten wurde. In dieser Literaturstelle wird CAU-10-H in einem 37 ml Autoklaven hergestellt. Zwar wird erwähnt, dass eine Maßstabsvergrößerung in größeren Autoklaven denkbar ist, der Nachweis wird aber nicht erbracht.

Gerade wasserstabile MOFs, die als Sorptionsmaterial für Wärmetransformationsanwendungen eingesetzt werden sollen, werden mit Wasser bei Überdruck hergestellt, was mit den bekannten, großtechnischen Problemen einhergeht:
1. Aufgrund der typischen Reaktionstemperaturen (>100 °C) muss unter Überdruck und in entsprechenden Gefäßen (Autoklaven) gearbeitet werden.
2. Dies erschwert die Reaktionskontrolle (kein Einblick in die Gefäße) und erhöht den Kostenaufwand enorm, insbesondere dann, wenn Solvothermalsynthesen verwendet werden sollen.
3. Die Verwendung von Glaskolben ist kaum oder nur eingeschränkt möglich.

Für MIL-160, einem zu CAU-10-H isostrukturellen MOF, ist eine drucklose Synthese in wässriger Lösung bekannt geworden, bei der Furandicarbonsäure mit Aluminium(III)chlorid über einen Zeitraum von 24 Stunden umgesetzt wird. Die Aufreinigung erfolgt mittels Zentrifugation. Nachteilig ist einerseits die Verwendung von Aluminium(III)chlorid, welches nicht wasserstabil und korrosiv ist. Die Aufreinigung mittels Zentrifugation ist zeitintensiv und apparativ aufwändig.

DE 10 2014 215 568 A1 offenbart ein Verfahren zur Herstellung eines Adsorbens aus metallorganischen Gerüststrukturen. Die Strukturen sollen bei Atmosphärendruck herstellbar sein. Es wird ein Lösungsmittelgemisch aus DMSO und Wasser verwendet, wobei relativ wenig Wasser und viel DMSO (wenigstens 50 Gew.-%) eingesetzt wird. Ziel ist es, durch das DMSO einen Siedepunkt oberhalb von 100°C zu erreichen. Das Wasser im Reaktionsgemisch spielt eine Nebenrolle und DMSO wird als entscheidend für den erfindungsgemäßen Erfolg angesehen. DMSO hat den Nachteil, dass es mit einigen Metallsalzen, die ebenfalls bei der MOF-Synthese eingesetzt werden, explosive Gemische bildet. Die Reaktionszeiten liegen im Bereich von 24 Stunden und sind damit im Vergleich zur vorliegenden Erfindung sehr lang. Das Dokument offenbart nicht die Verwendung einer wässrigen Lösung für die Synthese.

US 2011/0282071 A1 offenbart fotoaktive Triazolstrukturen. Es wird ein Beispiel für eine aromatische Dicarbonsäure gegeben. Es wird aber nicht die Synthese der aromatischen Dicarbonsäure in wässriger Lösung offenbart.

DE 10 2006 043 648 A1 lehrt ein Verfahren zur Herstellung von MOFs als Adsorbens. Die Synthese wird in einem organischen Lösungsmittel mit vergleichsweise hohem Siedepunkt durchgeführt, z. B. in DMF. Die Reaktionszeit liegt im Bereich von 5 Tagen.

WO 2013/186542 A1 beschreibt ein Verfahren zur Herstellung von MOFs, bei dem Benzoldicarboxylate mit Metallsalzen in wässriger Lösung unter Umgebungsdruck umgesetzt werden. Als Carbonsäuresalze werden für die Linker 2,5-Dihydroxyterephthalate (gerade Linker) und 1,3,5-Benzoltricarbonsäuresalze (verzweigende Linker) vorgestellt, die mit Salzmischungen aus Zn / Na oder Ni / Na ungesetzt werden. Hinweise auf V-förmige Linker allgemein oder Isophthalate und deren Derivate im Besonderen sowie Aluminiumsalze finden sich nicht. DE 10 2005 039 654 A1 betrifft mesoporöse MOF-Verbindungen. Es wird als entscheidend beschrieben, dass die strukturellen Merkmale wenigstens eines Stickstoffatoms im Heteroaromaten des Linkers und wenigstens dreier Substituenten X in Form von Carboxylgruppen (bzw. deren Thioderivaten) unbedingt einzuhalten sind. Andernfalls würden die hohen spezifischen Oberflächen und die gewünschte mesoporöse Struktur nicht erreicht. Es wird somit von der Verwendung aromatischer Dicarbonsäuren abgeraten. Die Synthese findet in organischem Lösungsmittel statt. Der Einsatz von Wasser wird nicht empfohlen. Die Reaktionszeiten sind mit 4 Tagen sehr lang. Es wäre ein Herstellungsverfahren für MOFs wünschenswert, das
- im Vergleich zu den Verfahren aus dem Stand der Technik weniger Reaktionszeit erfordert,
- im Hinblick auf die Umwelt unbedenklich ist,
- keine besonderen Anforderungen an die Arbeitssicherheit stellt (z. B. Explosionsgefahr),
- keinen großen apparativen Aufwand erfordert (z. B. Autoklav) und
- MOFs in sehr guter Qualität, insbesondere mit hoher Wasserstabilität und großer spezifischer Oberfläche, erhältlich macht.

Aufgabe der vorliegenden Erfindung war es daher, eine Syntheseroute für CAU-10-H und strukturell verwandte MOFs bereitzustellen, die die Nachteile des Standes der Technik überwindet.

Diese Aufgabe wird gelöst durch ein anspruchsgemäßes Verfahren zur Herstellung einer metallorganischen Gerüststrukturverbindung, bei dem mindestens ein Metallsalz umfassend ein Metallkation ausgewählt aus der Gruppe bestehend aus den Übergangsmetallen und Al sowie Kombinationen daraus mit einer Linkerverbindung umgesetzt wird, wobei die Umsetzung in wässriger Lösung bei einem Druck von 1,5 bar oder weniger erfolgt. Dabei ist die Linkerverbindung ausgewählt aus der Gruppe bestehend aus substituierten und unsubstituierten Isophthalaten sowie Kombinationen und Mischungen daraus.

Unter Isophthalaten oder Derivaten davon werden erfindungsgemäß Strukturen der allgemeinen Formel 2 verstanden.

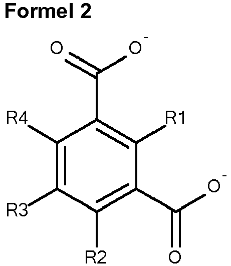

Die Reste R1 bis R4 sind, vorzugsweise unabhängig voneinander, ausgewählt aus Wasserstoff, Hydroxy-, Nitro-, Amino-, Methyl-, Ether- und Halogenidgruppen sowie Kombinationen davon. In bevorzugten Ausführungsformen sind alle Reste R1 bis R4 Wasserstoff. In bevorzugten Ausführungsformen ist R3 ausgewählt aus Amino-, Nitro-, Hydroxy-, Methylether- und Methylgruppe.

In erfindungsgemäßen Ausführungsformen ist die Linkerverbindung ausgewählt aus Isophthalaten. Erfindungsgemäß umfasst der Begriff "Isophthalate" auch deren an 2-, 4-, 5- oder 6-Position substituierte Derivate. Als Substituenten kommen Hydroxy-, Nitro-, Amino-, Methyl-, Ether- und Halogenidgruppen sowie Kombinationen davon in Frage.

Erfindungsgemäß findet die Reaktion in wässriger Lösung statt. Da viele aromatische Dicarbonsäuren in Wasser mitunter schlecht löslich sind, ist es erfindungsgemäß die Isophthalate zu verwenden. Die Isophthalatewerden in Form ihrer Salze, vorzugsweise als Natrium, Kalium oder Ammoniumsalze, eingesetzt.

Bevorzugte Metalle sind Fe, Co, Ni, Zn, Zr, Cu, Cr, Mo, Mn, Al, Pd und Kombinationen davon, besonders bevorzugt sind Al, Fe, Cu, Cr, Zr sowie Kombinationen davon. In ebenfalls bevorzugten Ausführungsformen sind die Metalle ausgewählt aus Sc, Ti, V, Cr, Mn, Fe, Co, Ni, Cu, Zn sowie Kombinationen davon. In besonders bevorzugten Ausführungsformen ist das Metall ausgewählt aus Al und Fe. Gerade bei Verwendung von Aluminium lassen sich sehr wasserstabile Gerüststrukturverbindungen herstellen. Die Metalle werden vorzugsweise in Form ihrer wasserlöslichen Salze, insbesondere der Sulfate, Nitrate, Carbonate, Oxidchloride oder Halogenide eingesetzt. Besonders bevorzugt ist die Verwendung von Sulfaten und/oder Halogeniden. Es kommen aber auch jeweils andere Salze in Frage.

"Umsetzung in wässriger Lösung" meint erfindungsgemäß bevorzugt, dass im Wesentlichen keine organischen Lösungsmittel in dem Reaktionsmedium eingesetzt werden. Vorteilhafte wässrige Lösungen umfassen weniger als 10 Vol.-%, insbesondere weniger als 5 Vol.-%, weiter bevorzugt weniger als 2 Vol.-% und besonders bevorzugt weniger als 1 Vol.-% organisches Lösungsmittel wie DMF. In bevorzugten Ausführungsformen enthält das Reaktionsmedium keinerlei organisches Lösungsmittel. Der Anteil an Wasser in der wässrigen Lösung beträgt insbesondere mehr als 50 Vol.-%, mehr bevorzugt wenigstens 70 Vol.-%, insbesondere mehr als 80 Vol.-% und besonders bevorzugt wenigstens 90 Vol.-% oder wenigstens 99 Vol.-%.

In besonderen Ausführungsformen können kurzkettige Alkohole mit Kohlenstoffkettenlängen von 1 bis 4 Kohlenstoffatome, insbesondere Ethanol, in den Reaktionsmedien dieser Erfindung eingesetzt werden. Deren Anteil ist vorzugsweise auf höchstens 20 Vol.-%, mehr bevorzugt höchstens 10 Vol.-% des verwendeten Lösungsmittels beschränkt.

Kernpunkt der vorliegenden Erfindung ist ein neuer Syntheseansatz, bei dem abweichend vom Stand der Technik eine im Wesentlichen drucklose Synthese durchgeführt wird. Die vorliegende Erfindung umfasst vorzugweise als weiteren Aspekt die Verwendung von Lösungen der Edukte und nicht von Feststoffen bzw. Suspensionen. Dies ist insbesondere in Kombination mit der drucklosen Synthese von Vorteil. So kann als Lösungsmittel vorzugsweise Wasser eingesetzt werden, und es ist kein Druckreaktor notwendig.

Daher erfolgt in einer bevorzugten Ausgestaltung des erfindungsgemäßen Verfahrens die Umsetzung bei einem Druck von wenigstens 900 mbar, insbesondere wenigstens 1 bar. In bevorzugten Ausführungsformen beträgt der Druck höchstens 1,2 bar oder höchstens 1,1 bar. Die Reaktion kann also bei Atmosphärendruck durchgeführt werden. Hieraus ergeben sich unmittelbare wirtschaftliche Vorteile bei der großtechnischen Umsetzung des Herstellungsverfahrens. Beispielsweise kann eine kontinuierliche Produktion realisiert werden, indem entstandenes Produkt aus dem Prozess entnommen wird. Dies kann durch Filtration erfolgen. Eine vorteilhafte Ausgestaltung des erfindungsgemäßen Verfahrens umfasst die Isolierung der hergestellten metallorganischen Gerüststrukturverbindung mittels Filtration. Isolierung des Produktes mittels Filtration ist leichter in ein kontinuierliches Verfahren umzusetzen als die Isolierung mittels Zentrifugation.

Weiterhin ist die Aufarbeitung wesentlich einfacher, da nur ein Waschschritt notwendig ist und keine thermische Aktivierung, um beispielsweise Reste von DMF zu entfernen.

Im Ergebnis wird die Recyclingfähigkeit dadurch deutlich erhöht, und die Reste des Syntheseansatzes können ohne weitere Nachbehandlung direkt der Kläranlage zugeführt werden. Auch das Produkt ist somit unmittelbar frei von organischem Lösungsmittel.

In einer bevorzugten Ausgestaltung des erfindungsgemäßen Verfahrens erfolgt die Umsetzung bei einer Temperatur von 80 bis 120°C, insbesondere 90 bis 110°C. In einer Ausführungsform beträgt die Umsetzungstemperatur maximal 100°C. Die Umsetzung erfolgt insbesondere am Siedepunkt des Reaktionsmediums.

Ein weiterer technischer Vorteil der erfindungsgemäßen Synthesemethode liegt darin, dass verglichen mit der schlechten Löslichkeit der aromatischen Dicarbonsäure (Isophthalsäure) das aromatische Dicarboxylat (Isophthalat) leicht in Wasser gelöst wird. Das hat insbesondere für eine industrielle Umsetzung Vorteile, da die Reaktionszeit deutlich verkürzt wird, von 12 h wie in der Literatur auf beispielsweise 6 h oder 3 h im Kolben. Damit einhergehend kann ein höherer STY (Raum-Zeit-Ausbeute) erreicht werden. Daher erfolgt in einer bevorzugten Ausgestaltung des erfindungsgemäßen Verfahrens die Umsetzung über einen Zeitraum von 10 Stunden oder weniger, insbesondere von 8 Stunden oder weniger, besonders bevorzugt 6 Stunden oder weniger.

In einer weiteren bevorzugten Ausgestaltung des erfindungsgemäßen Verfahrens erfolgt die Umsetzung unter Bestrahlung der wässrigen Lösung mit Mikrowellen. Es sind aber auch andere dem Fachmann geläufige Verfahren zur Erwärmung des Reaktionsgefäßes erfindungsgemäß.

Im Gegensatz zur literaturbekannten Synthese wird gemäß bevorzugten erfindungsgemäßen Verfahren von aromatischen Dicarboxylaten (Isophthalaten) ausgegangen und nicht von der in Wasser schwer löslichen aromatischen Dicarbonsäure (Isophthalsäure). In der literaturbekannten Synthese muss daher wegen der schlechten Löslichkeit DMF als Lösungsmittel und erhöhte Temperatur eingesetzt werden.

Für das erfindungsgemäße Verfahren kann jedes beliebige Isophthalat verwendet werden. Bevorzugt verwendet werden Natriumisophthalate, Kaliumisophthalate, Ammoniumisophthalate und Mischungen daraus.

Für das erfindungsgemäße Verfahren können prinzipiell alle zuvor beschriebenen Metallsalze verwendet werden. Vorzugsweise verwendet werden Eisen- und Aluminiumsalze.

Im Gegensatz zur literaturbekannten Synthese wird vorzugsweise Aluminium-Sulfat in Kombination mit Natriumdicarboxylat (z. B. Natriumisophthalat) sowie gleichzeitig eine anorganische Base, insbesondere Natriumhydroxid, Calciumhydroxid, Kaliumhydroxid, Ammoniak oder Natriumaluminat in einer Lösung und in einem Glasgefäß anstelle eines Teflongefäßes verwendet.

Die Verwendung von Al-Sulfat ist zunächst nicht naheliegend, da die Bildung von Nebenphasen, insbesondere Alunit (KAl₃[(OH)₆(SO₄)₂]), deutlich verstärkt auftritt. Um die Bildung dieser Nebenphasen zu unterbinden bzw. zu minimieren wird vorzugsweise Kaliumhydroxid, Natriumhydroxid, Calciumhydroxid, Ammoniak oder Natriumaluminat als Base zugesetzt. Natriumaluminat wird dabei als kombinierte Metallquelle und Base benutzt, was aus der Literatur nicht bekannt ist. Dementsprechend wird in einer bevorzugten Ausgestaltung des erfindungsgemäßen Verfahrens als Metallsalz Aluminiumsulfat eingesetzt. In einer weiteren bevorzugten Ausgestaltung des erfindungsgemäßen Verfahrens wird der wässrigen Lösung eine Base zugesetzt. Vorzugsweise ist die Base ausgewählt aus der Gruppe bestehend aus Ammoniak, Natriumhydroxid, Kaliumhydroxid, Natriumaluminat und Kaliumaluminat. Besonders bevorzugt ist Natriumaluminat.

In einer alternativen bevorzugten Ausgestaltung des erfindungsgemäßen Verfahrens wird als Metallsalz Eisen(III)-chlorid verwendet.

Die mit dem erfindungsgemäßen Verfahren hergestellten metallorganischen Gerüststrukturverbindungen zeichnen sich durch besonders hohe Wasserbeständigkeit aus. Vorzugsweise weist die metallorganische Gerüststrukturverbindung eine spezifische Oberfläche nach BET von 500 m²/g oder mehr auf.

Eine Verwendung finden diese metallorganischen Gerüststrukturverbindungen als Adsorbens, wobei der adsorbierte Stoff (Adsorbat) vorzugsweise Wasser, Ethanol, Methanol, Methan, CO₂, H₂ oder eine Mischung daraus ist. Bevorzugt ist insbesondere die Verwendung der hierin beschriebenen metallorganischen Gerüststrukturverbindungen für Anwendungen wie Gasspeicherung, Katalyse, Entfeuchtung und Wärmetransformation (z. B. Wärmepumpen, Kältemaschinen).

Mit dem erfindungsgemäßen Verfahren lassen sich hohe Ausbeuten von über 90 % bezogen auf die Linkerverbindung erzielen. Die mit diesem Verfahren hergestellten metallorganischen Gerüststrukturverbindungen zeigen die gleichen oder größere Oberflächen, die gleichen oder größere Kapazitäten hinsichtlich Gassorption und damit die gleichen oder bessere technische Eigenschaften als nach einem Verfahren des Standes der Technik hergestellte metallorganische Gerüststrukturverbindungen. Die metallorganischen Gerüststrukturverbindungen, die durch Reaktion in wässriger Lösung hergestellt wurden, zeichnen sich ferner durch die Abwesenheit von Rückständen organischer Lösungsmittel aus.

Die vorliegende Erfindung wird anhand der nachfolgenden Beispiele näher erläutert.

### Beispiele

Für die zu untersuchenden Proben wurde zu Beginn der zyklenunabhängigen Alterung eine Eingangsmessung mit Stickstoff (N₂) bei 77 Kelvin an einer NOVA 3000e der Fa. Quantachrome durchgeführt. Über die Stickstoffmessung bei 77 Kelvin lassen sich Aussagen über die Veränderung der Porenstruktur (Porenradienverteilung), Porenvolumen sowie über die innere Oberfläche (BET) treffen. Um die Proben von Feuchte und Fremdgasen zu befreien, wurden diese vor der eigentlichen Messung im Hochvakuum für 24 h bei 120 °C ausgeheizt. Danach wurde das Trockengewicht der Probe mit einer Analysenwaage der Fa. Sartorius mit der Genauigkeitsklasse I bestimmt. Anschließend wurden komplette Isothermen in Ad- und Desorption aufgenommen und ausgewertet. Der Relativ-Druckbereich wurde zwischen p/p₀ = 0,05-0,999 in Adsorption und p/p₀ =0,999-0,1 in Desorption gefahren. Das Porenvolumen wurde nach der Dichte Funktional Theorie (DFT) und nach dem Modell von Dubinin und Astakhov (DA) bestimmt. Die innere Oberfläche wurde nach dem Modell von Brunauer-Emmett-Teller (BET) zwischen p/p₀ = 0,05 und 0,15 bestimmt.

### Vergleichsbeispiel 1

Synthese: 200 mg 1,3-Isophthalsäure (1,3-H₂BDC, 1,20 mmol), gelöst in 1 mL N,N-Dimethylformamid (DMF), wurden mit 800 mg Al₂(SO₄)₃*18 H₂O, gelöst in 4 mL H₂O, gemischt und in einem mit Teflon ausgekleideten Stahlautoklaven für 12 Stunden bei 135 °C behandelt.

Aufarbeitung: Nach Abkühlenlassen auf Raumtemperatur wurde das Produkt filtriert und im Ultraschallbad mit Wasser gewaschen. Der erhaltene weiße Feststoff wurde getrocknet und danach für 24 Stunden bei 120 °C im Vakuum aktiviert.

Die spezifische Oberfläche des Produktes betrug S_{BET} = 525 m²/g und das Porenvolumen lag bei 0,27 cm³/g.

### Vergleichsbeispiel 2

Synthese: Eine Lösung von 0,75 mol (125 g) Isophthalsäure in 600 ml DMF und 2400 ml Wasser und 0,72 mmol (483 g) Al₂(SO₄)₃*18 H₂O wurde im 5000-ml-Dreihalskolben auf 135 °C erwärmt.

In einem 5 L-Kolben wurde 483 g (0,72 mol) Al₂(SO₄)₃*18 H₂O in 2,4 L Wasser vollständig gelöst. Zu der Aluminiumsulfat-Lösung wurde 125 g (0,75 mol) Isophthalsäure gelöst in 600 mL DMF portionsweise zugegeben.

Die Lösung wurde für eine Dauer von 48 h unter Rühren refluxiert.

Aufarbeitung: Der entstandene Feststoff wurde über einen Faltenfilter (5 - 13 µm) abfiltriert, in H₂O resuspendiert und für 30 Minuten in ein Ultraschallbad gestellt. Dieser Vorgang wurde 3 Mal wiederholt. Anschließend wurde der weiße Feststoff 5 Tage bei 90 °C im Trockenschank und 1 Tag bei 120 °C im Vakuumschrank getrocknet.

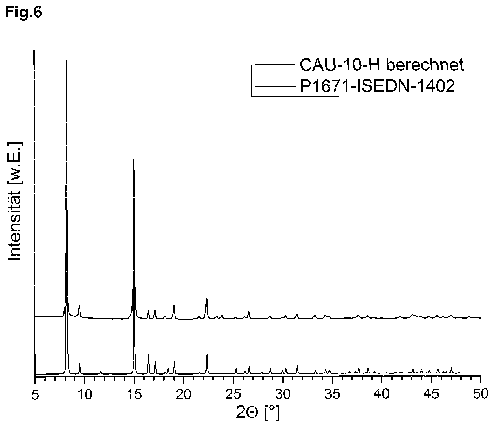

Nach der Aufreinigung erhielt man 156,8 g eines weißen Feststoffs mit S_{BET} = 578 m²/g. Die einzige kristalline Phase wurde mittels Röntgen-Pulverdiffraktometrie als CAU-10-H identifiziert. Figur 6 zeigt das Pulverdiffraktogramm von CAU-10-H.

### Ausführungsbeispiel 1

Synthese: Es wurden 5 L einer 0,5 M Natriumisophthalat-Lösung hergestellt, indem Natriumhydroxid (199,99 g; 5 mol) und Isophthalsäure (415,33 g; 2,5 mol) in einem Messkolben mit H₂O auf 5000 mL aufgefüllt wurden. Des Weiteren wurden je 2 L einer 0,5 M Aluminiumsulfat*18 H₂O-Lösung (666,15 g; 1 mol) und 2 L einer 0,5 M Natriumaluminatlösung (81,79 g; 1 mol), jeweils durch Auffüllen im Messkolben mit H₂O auf 2000 mL, hergestellt. Für die Reaktion wurden 2,16 L Natriumisophthalat-Lösung (0,5 M) und 180 mL Ethanol vorgelegt und unter Rühren 810 mL Aluminiumsulfatlösung (0,5 M) und 540 mL Natriumaluminatlösung (0,5 M) zugegeben. Die Reaktion wurde danach für 6 h unter Rückfluss und Rühren durchgeführt.

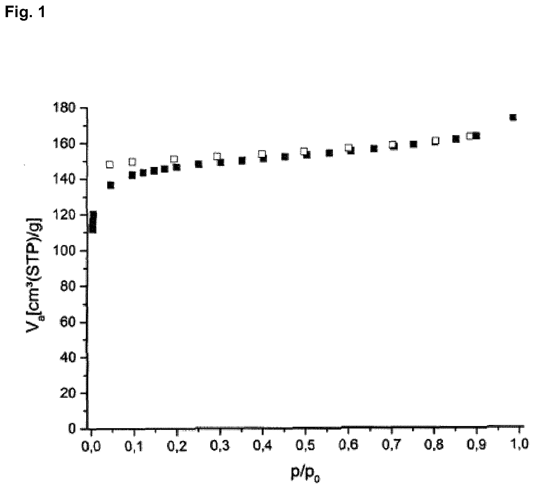

Aufarbeitung: Der erhaltene Feststoff wurde abfiltriert, mit reichlich Wasser und Ethanol gewaschen und über Nacht bei 90 °C getrocknet. Es wurden 207 g (92 % Ausbeute) weißer pulverförmiger Feststoff (S_{BET} = 580 m²/g) erhalten, welcher mittels Röntgenpulverdiffraktometrie als CAU-10-H identifiziert wurde. Die N₂-Sorptionsisotherme ist in Fig. 1 gezeigt; ausgefüllte Quadrate beschreiben die Adsorptionskurve und leere Quadrate beschreiben die Desorptionskurve.

### Ausführungsbeispiel 2

Synthese: Für die Synthese wurden 100 mL einer 0,5 M Natriumisophthalat-Lösung erstellt, indem Natriumhydroxid (3,99 g, 0,1 mol) und Isophthalsäure (8,30 g; 0,05 mol) in einem Messkolben mit H₂O auf 100 mL aufgefüllt wurden. Des Weiteren wurden je 100 mL einer 0,5 M Aluminiumsulfat*18 H₂O-Lösung (33,308 g; 0,05 mol) und 100 mL einer 2 M Natriumhydroxidlösung (7,99 g; 0,2 mol), jeweils durch Auffüllen im Messkolben mit H₂O auf 100 mL, hergestellt. Für die Reaktion wurden 127,5 mL H₂O, 7,5 mL Ethanol und 90 mL Natriumisophthalat-Lösung vorgelegt und unter Rühren 45 mL Aluminiumsulfatlösung und 22,5 mL Natriumhydroxidlösung zugegeben. Die Reaktion wurde danach für 6 h unter Rückfluss und Rühren durchgeführt.

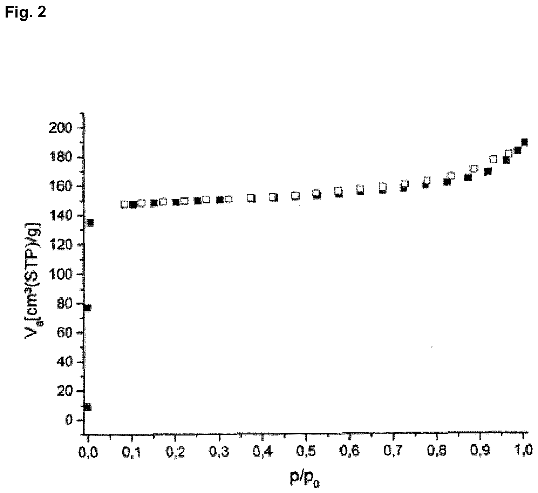

Aufarbeitung: Der erhaltene Feststoff wurde abfiltriert, mit reichlich Wasser und Ethanol gewaschen und über Nacht bei 100 °C getrocknet. Es wurde ein pulverförmiger Feststoff (S_{BET} = 573 m²/g) erhalten, welcher mittels Röntgenpulverdiffraktometrie als CAU-10-H identifiziert wurde. Des Weiteren enthält das Reaktionsprodukt eine Nebenphase (Natronalunit, NaAl₃(OH)₆(SO₄)₂; #(ICSD) = 44626). Die N₂-Sorptionsisotherme ist in Fig. 2 gezeigt; ausgefüllte Quadrate beschreiben die Adsorptionskurve und leere Quadrate beschreiben die Desorptionskurve.

### Ausführungsbeispiel 3

Synthese: 5,25 mL einer 0,5 M Natriumisophthalat-Lösung wurden mit 4,5 mL Wasser verrührt. Unter Rühren wurden 5,25 mL einer 0,5 M FeCl₃-Lösung hinzugefügt. Die Reaktion wurde danach für 6 h bei 95 °C in der Mikrowelle unter Rühren durchgeführt.

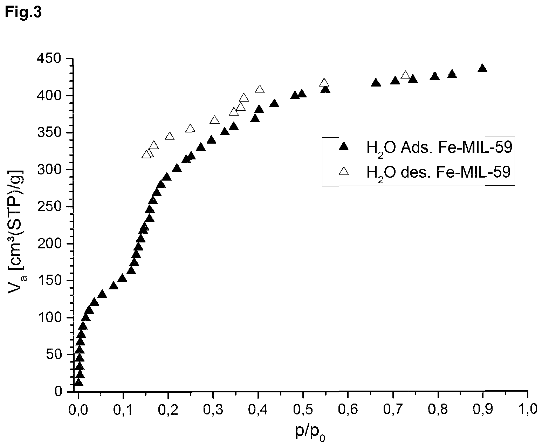

Aufarbeitung: Der erhaltene Feststoff wurde abfiltriert, mit reichlich Wasser und Ethanol gewaschen und über Nacht bei 90 °C getrocknet. Er konnte als Fe-MIL-59 identifiziert werden. Figur 3 zeigt die mit diesem Stoff erhaltene Wasser-Sorptionsisotherme.

### Ausführungsbeispiel 4

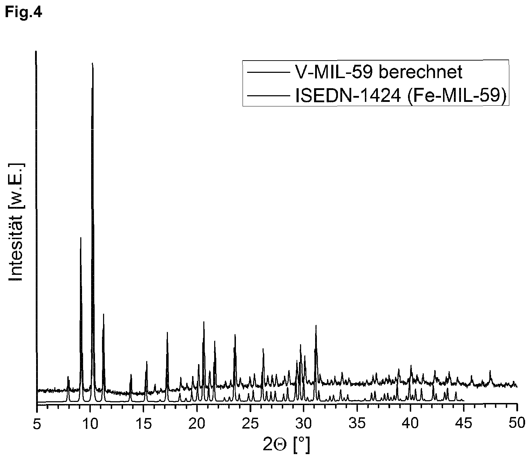

Für eine weitere Synthese von Fe-MIL-59 wurden 100 mL m-Na₂-BDC-Lösung (0,5 M) und 50 mL Wasser vorgelegt und unter Rühren 100 mL FeCl₃-Lösung (0,5 M) zugegeben. Die Reaktion wurde unter starkem Rühren und Rückfluss für 6 Stunden durchgeführt. Der erhaltene Feststoff wurde über einen sehr feinen Filter abfiltriert und der Feststoff gründlich mit Wasser gewaschen. Das Produkt wurde für 3 Tage im Trockenofen (90 °C) getrocknet. Es wurde ein orangebrauner Feststoff erhalten. Die Ausbeute beträgt 12,55 g (max. 12.8 g, 98 %). Das gemessene Pulverdiffraktogramm ist in Figur 4 dargestellt. Als Vergleich dient das Diffraktogramm von Vanadium-MIL-59 welches isostrukturell ist.

### Vergleichsbeispiel 3

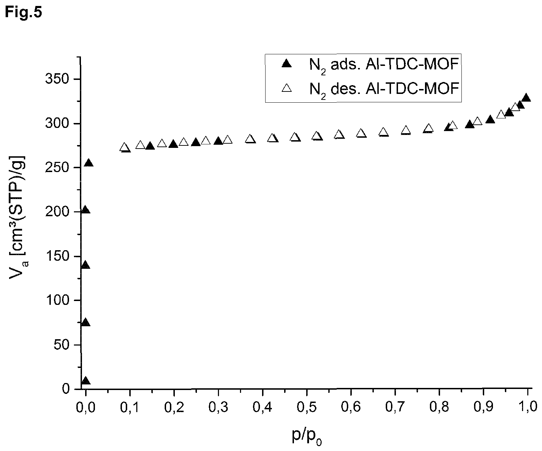

Es wurden 7,5 mL Na₂TDC-Lösung (0,5 M) vorgelegt und unter Rühren 5,625 mL AlCl₃-Lösung (0,5 M) und 1,875 mL NaAlO₂-Lösung (0,5 M) zugegeben. Die Reaktion fand für 3 h bei 95 °C unter Rühren in der Mikrowelle statt. Der erhaltene Feststoff wurde filtriert und mit Wasser und Ethanol gewaschen. Eine Analyse ergab eine Oberfläche (BET) von 1024 m²/g und ein Porenvolumen = 0,4381 cm³/g. Figur 5 zeigt die N₂-Sorptionsisotherme.

Aus dem Vergleich von Vergleichsbeispiel 2 mit den Ausführungsbeispielen 1 bzw. 2 ist ersichtlich, dass die Verwendung von Isophthalaten in wässrigen Reaktionsmedien die Reaktionszeit wesentlich verkürzt.

Ausführungsbeispiel 3 zeigt, dass die Reaktion nicht nur mit Aluminium als Metallkomponente funktioniert.

Vergleichsbeispiel 3 zeigt, dass die Reaktion in analoger Weise mit anderen aromatischen Dicarbonsäuren durchführbar ist.

## Patentansprüche

1. Verfahren zur Herstellung einer metallorganischen Gerüststrukturverbindung, bei dem mindestens ein Metallsalz umfassend ein Metallkation ausgewählt aus der Gruppe bestehend aus den Übergangsmetallen und Al sowie Kombinationen daraus mit einer Linkerverbindung umgesetzt wird, wobei die Umsetzung bei einem Druck von weniger als 1,5 bar in wässriger Lösung erfolgt, **dadurch gekennzeichnet, dass** die Linkerverbindung ein Isophthalat oder ein Derivat davon ist, wobei die Linkerverbindung eine Struktur gemäß der allgemeinen Formel 2 aufweist: 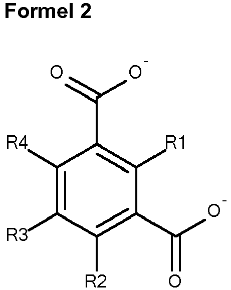 wobei die Reste R1 bis R4 unabhängig voneinander ausgewählt sind aus Wasserstoff, Hydroxy-, Nitro-, Amino-, Methyl-, Ether- und Halogenidgruppen sowie Kombinationen davon.

2. Verfahren nach Anspruch 1, **dadurch gekennzeichnet, dass** die wässrige Lösung weniger als 10 Vol.-% organischen Lösungsmittels aufweist.

3. Verfahren nach Anspruch 1 oder 2, **dadurch gekennzeichnet, dass** die Umsetzung bei einer Temperatur 80 bis 120°C oder mehr erfolgt.

4. Verfahren nach einem der Ansprüche 1 bis 3, **dadurch gekennzeichnet, dass** die Umsetzung am Siedepunkt des Reaktionsmediums erfolgt.

5. Verfahren nach einem der Ansprüche 1 bis 4, **dadurch gekennzeichnet, dass** die Umsetzung über einen Zeitraum von 10 Stunden oder weniger erfolgt.

6. Verfahren nach einem der Ansprüche 1 bis 5, **dadurch gekennzeichnet, dass** das Metallsalz ausgewählt ist aus der Gruppe bestehend aus Eisen- und Aluminiumsalzen.

7. Verfahren nach einem der Ansprüche 1 bis 6, **dadurch gekennzeichnet, dass** der wässrigen Lösung eine Base zugesetzt wird.

## Claims

1. A method for the production of a metal-organic framework compound, in which at least one metal salt comprising a metal cation selected from the group consisting of the transition metals and Al as well as combinations thereof is reacted with a linker compound, wherein the reaction is conducted at a pressure of less than 1.5 bar in aqueous solution, **characterized in that** the linker compound is an isophthalate or a derivate thereof, wherein the linker compound has a structure according to the general formula 2: 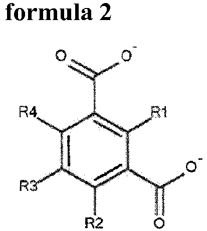 wherein the groups R1 to R4 are independently from each other selected from hydrogen, hydroxyl, nitro, amino, methyl, ether and halide groups as well as combinations thereof.

2. The method according to claim 1, **characterized in that** the aqueous solution comprises less than 10 % by volume of organic solvent.

3. The method according to claim 1 or 2, **characterized in that** the reaction is conducted at a temperature of 80 to 120°C or higher.

4. The method according to one of claims 1 to 3, **characterized in that** the reaction is conducted at the boiling point of the reaction medium.

5. The method according to one of claims 1 to 4, **characterized in that** the reaction is conducted over a period of time of 10 hours or shorter.

6. The method according to one of claims 1 to 5, **characterized in that** the metal salt is selected from the group consisting of iron and aluminum salts.

7. The method according to one of claims 1 to 6, **characterized in that** a base is added to the aqueous solution.

## Revendications

1. Procédé de préparation d'un composé de réseau organométallique, procédé dans lequel au moins un sel métallique, comprenant un cation métallique choisi dans le groupe comprenant des métaux de transition et l'Al et leur combinaison, est mis à réagir avec un composé de liaison, dans lequel la réaction se produit à une pression inférieure à 1,5 bar dans une solution aqueuse, **caractérisé en ce que** le composé de liaison est un isophtalate ou un dérivé de celui-ci, le composé de liaison ayant une structure correspondant à la formule générale 2 : Formule 2 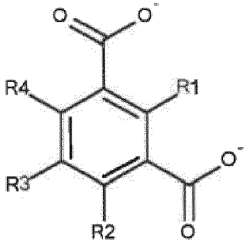 les radicaux R1 à R4 étant choisis indépendamment l'un de l'autre parmi l'hydrogène et les groupes hydroxy, nitro, amino, méthyl, éther et halogénure et leurs combinaisons.

2. Procédé selon la revendication 1, **caractérisé en ce que** la solution aqueuse comporte moins de 10 % en volume de solvant organique.

3. Procédé selon la revendication 1 ou 2, **caractérisé en ce que** la réaction se produit à une température de 80 à 120 °C ou plus.

4. Procédé selon l'une des revendications 1 à 3, **caractérisé en ce que** la réaction se produit à la température d'ébullition du milieu réactionnel.

5. Procédé selon l'une des revendications 1 à 4, **caractérisé en ce que** la réaction se produit sur une durée de 10 heures ou moins.

6. Procédé selon l'une des revendications 1 à 5, **caractérisé en ce que** le sel métallique est choisi dans le groupe comprenant des sels de fer et d'aluminium.

7. Procédé selon l'une des revendications 1 à 6, **caractérisé en ce qu'**une base est ajoutée à la solution aqueuse.
